# EUROPEAN PATENT SPECIFICATION

(11) **EP 2 506 806 B1**
(45) Date of publication and mention of the grant of the patent: **17.04.2013**
(21) Application number: 11706178.8
(22) Date of filing: 18.02.2011
(51) Int. Cl.: A61F 2/36, A61F 2/30

(54) **FEMORAL NECK PROSTHESIS**
SCHENKELHALSPROTHESE
PROTHÈSE DE COL DU FÉMUR

(30) Priority: 25.02.2010 IT MI20100307
(43) Date of publication of application: 10.10.2012
(73) Proprietor: Permedica S.p.A., 23807 Merate (IT)
(72) Inventor: CAMERA, Andrea, I-17020 Calice Ligure (IT)
(74) Representative: Martegani, Franco
(86) International application number: PCT/EP2011/000860
(87) International publication number: WO 2011/104003

(56) References cited:
- EP-A1- 0 791 342
- WO-A1-03/039411
- WO-A1-03/047471
- DE-C1- 19 720 493
- DE-U1-202004 016 353
- US-A1- 2008 200 990
- US-B1- 7 494 509

## Description

The present invention relates to a femoral neck prosthesis.

Femoral neck prostheses are prosthetic devices implanted into the neck of the femur and not involving metaphyseal and diaphyseal femoral structures, unlike the more conventional hip prosthesis stems which are fitted into the medullary canal of the femur, such as for example the one described in EP0290735.

Devices known as femoral neck prostheses are already on the market, such as the one described in US 2009/076619 A1, and their object is to preserve as much as possible the healthy parts of the femur to be subjected to prosthetization. This is made possible by replacing only the parts affected by degeneration due to arthrosis (usually the femoral head with reference to the femur). In short a minimized plant is desired allowing the region of the patient body involved in the implantation to be damaged as little as possible.

Such search for a small, mini-invasive, bone-saving and soft tissue-saving implant certainly is considerably advantageous for the patient in terms of a reduction of the surgical time. Consequently a reduction in blood losses, an early rehabilitation, a very simplified implant revision, if needed, are desired as well.

The characteristic of such devices is the total preservation of the femur neck, which consequently has to be healthy in order to act as the seat for anchoring and holding the device. The calcar region, placed in the lower part of the femur neck, due to its anatomical configuration, is particularly suitable for supporting loads transferred by the device to the bone, and it has to be absolutely preserved.

Even in this way such prostheses have not to be confused with conventional hip prosthesis stems which in order to be fitted into the medullary canal of the femur require the calcar bone to be considerably wasted. Such waste inevitably leads the calcar to be less able to support heavy loads, which have to be supported by other anatomical structures of the femur (cortical diaphyseal structures etc...).

To this end, in a femoral neck prosthesis, the femoral head has to be resected at the highest possible point, precisely a subcapital resection, unlike in hip prosthesis stems.

A further main characteristic required for this type of "small" prosthesis is the method for anchoring it to the bone which has to give the device an excellent primary and secondary stability.

A standard prosthesis, such as hip prosthesis stems, has a portion with a linear extension which is quite long and involves a meta-diaphyseal anchorage, based on the principle of a cone (the stem) fitted into a hollow cylinder (the medullary canal of the femur) which are fastened one another by a mutual interference when the outer dimensions of the cone reach those of the cylinder. In a very different manner, a femoral neck prosthesis, object of the present invention, cannot follow such principle, as it does not involve the region of the canal of the femur.

The region in which such devices are fitted is characterized by the presence of a main lower cortical region (calcar) and of an inner trabecular region, wherein the bone is arranged along particular load transmitting lines forming two series of arches, called trabeculae, representing the lines of force within the femoral neck and the proximal part of the femur.

A neck prosthesis has to take part in such anatomical situation in a harmonious manner, affecting as little as possible the biomechanics and therefore the load transmission from the femoral head to the distal part of the femur.

In this perspective, the stability of the implant is given by the perfect harmonization between the implant and the bony structure provided.

The neck prostheses on the market generally have a straight cylindrical shape or a curved "banana-like" shape. Straight (both conical and cylindrical) neck prostheses clearly affect the femoral biomechanics coming in conflict with the curved bone trabeculae, intersecting them and modifying the release of the forces.

Moreover, due to the fact that they are developed about a single straight axis they do not guarantee the rotational stability of the device.

With the aim of overcoming such drawback the curvilinear or "banana-like" curved shaped neck prostheses are more reliable and suitable, such as for example the device object of the document EP 1 709 944 A2.

Such device is firstly designed as a resurfacing prosthesis for the femoral head which has a curved central shaft attached thereto.

The femoral resection required for applying such device is not a subcapital resection, but it is a resection that can be defined as hemicephalic, which is more like the resections for resurfacing prostheses for the femoral head. The prosthesis preserves not only the neck, but also half of the head.

These choices lead to the following drawbacks:
- since the stem is an evolutionary development of the stem of the resurfacing prostheses it is too thin and small in order to guarantee the primary stability to the implant, even if it is curved and therefore biomechanically correct with reference to the release of forces;
- the fact that half of the head is preserved makes it necessary to apply heads with large diameter which "resurface" the remaining head portion. Consequently the acetabular component wherein the head is articulated will have a greater size, with a subsequent considerable waste of the acetabular bone;
- implants with heads having a large diameter are required to use metal-on-metal couplings, which can result in metallosis or can trigger allergies in patients affected by hypersensibility to metals contained into the alloy;
- the stability of the prosthesis is almost completely given to the remaining portion of the head. In this region, the device is anchored to the bone by bone cement. It is known that during the polymerization the cement results in reaching such temperatures to necrotize the underlying bone, with damages to the structure of the femoral head (which is already not much vascularised) that over time result in a periprosthetic bone resorption and consequently in an early implant loosening.

From patent documents DE 19720493 C1, DE 202004016353 U1 and WO 2003039411 A1, it's also known a femoral neck prosthesis comprising a frustoconical body positionable into a seat of a neck of a femur, a curved tail, extending from an end of said frustoconical body, first and second anti-rotation means in order to prevent the prosthesis from rotating on its axis in the operating condition, with the first anti-rotation means being substantially coinciding with the cited curved tail, and the second anti-rotation means being provided on the body.

The generic aim of the present invention is to overcome the above mentioned drawbacks of the prior art in a very simple, inexpensive and especially functional way.

A further aim of the present invention is to provide a femoral neck prosthesis showing an excellent anchorage to the bone.

A further aim of the present invention is to provide a femoral neck prosthesis able to be fitted in a very easy way, while achieving an excellent stability and while damaging the bone as little as possible.

A further aim of the present invention is to provide a femoral neck prosthesis able to effectively resist the torsional moments generated on the device due to the walking action and which tend to cause it to rotate about its axis leading to the immediate failure of the implant.

Not last aim of the present invention is that of providing a femoral neck prosthesis showing a great fixation over time, while avoiding any forced anchorage as much as possible.

Considering the above aims, according to the present invention, a femoral neck prosthesis showing the characteristics stated in the annexed claims has been suggested to be made.

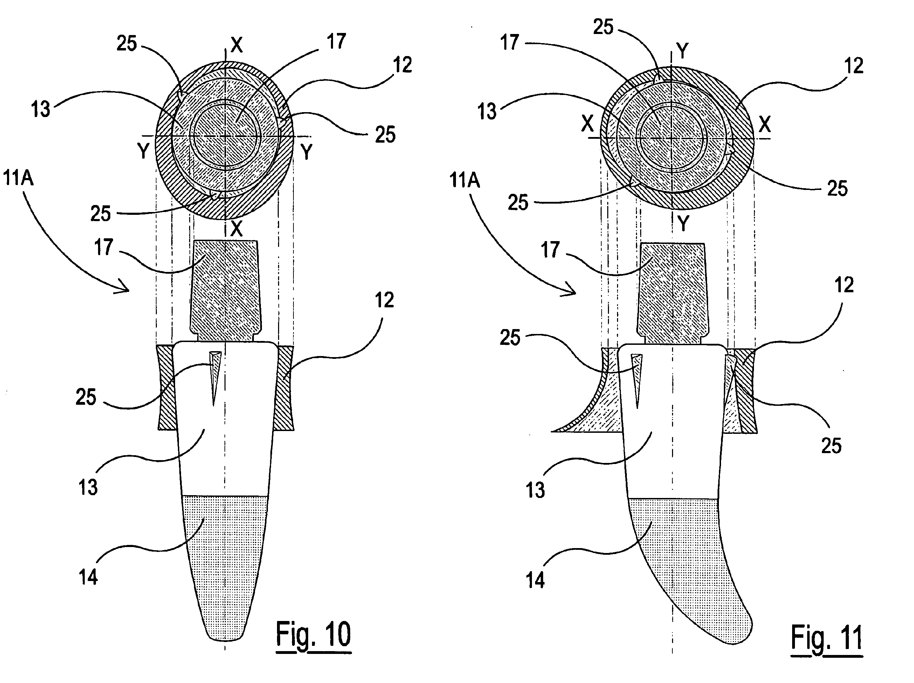

The structural and functional characteristics of the present invention and its advantages compared to the prior art will become even more clear and evident from an examination of the following description, referring to the attached drawings, wherein figures 10 and 11 show an embodiment of a femoral neck prosthesis made according to the invention itself.

In the drawings:
- figure 1 is a schematic side elevational view of a femoral neck prosthesis not falling under the invention;
- figure 2 is a schematic view showing the position in the femur of the prosthesis of figure 1 taken from a first angle (antero-posterior projection);

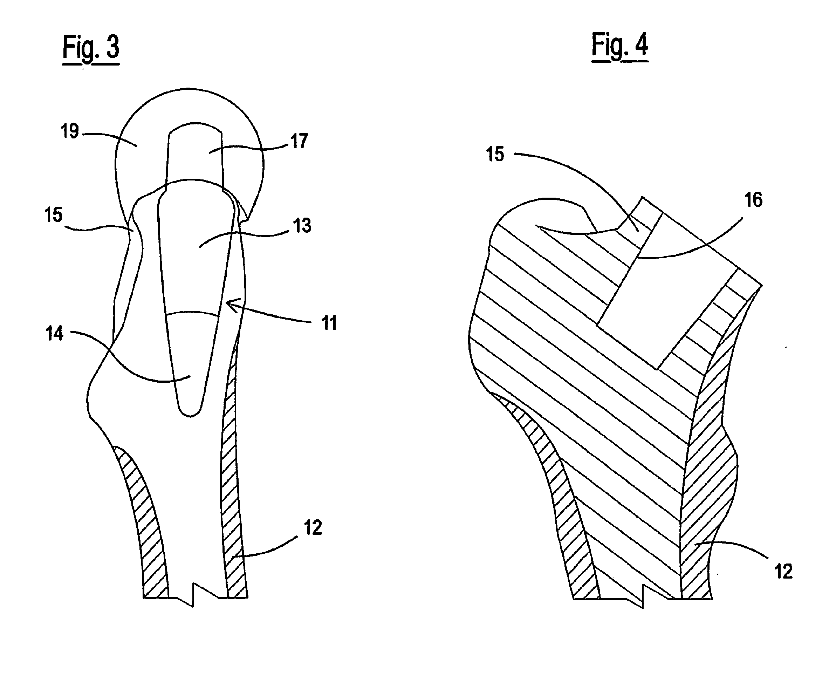

- figure 3 is a schematic view showing the position in the femur of the prosthesis of figure 1 taken from a second angle (medium-lateral projection);
- figures 4 and 5 are schematic views showing the femur before receiving the prosthesis of figure 1 and after fitting the prosthesis;
- figure 6 is a view of the femur with the prosthesis with a covering head arranged on a stump;

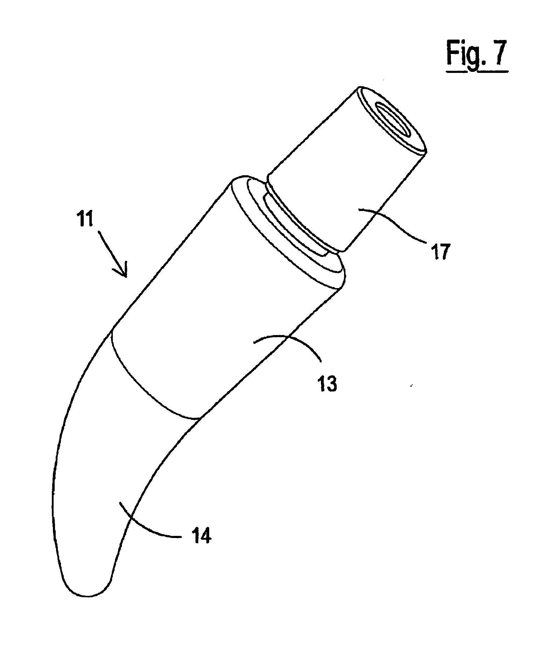

- figure 7 is a perspective view of the prosthesis,
- figure 8 is a view according to a first plane AA of the prosthesis of fig.1;
- figure 9 is a view according to a second plane BB of the prosthesis of fig.1;
- figure 10 is a view according to a first plane XX of a prosthesis according to the invention;
- figure 11 is a view according to a second plane YY of the prosthesis of fig.10;
- figure 12 is a plan view of a variant of the prosthesis of fig.1;
- figure 13 is a plan view of a variant of the prosthesis of fig.1;
- figure 14 is a plan view of a variant of the prosthesis of fig.1;
- figure 15 is a plan view of a variant of the prosthesis of fig.1;

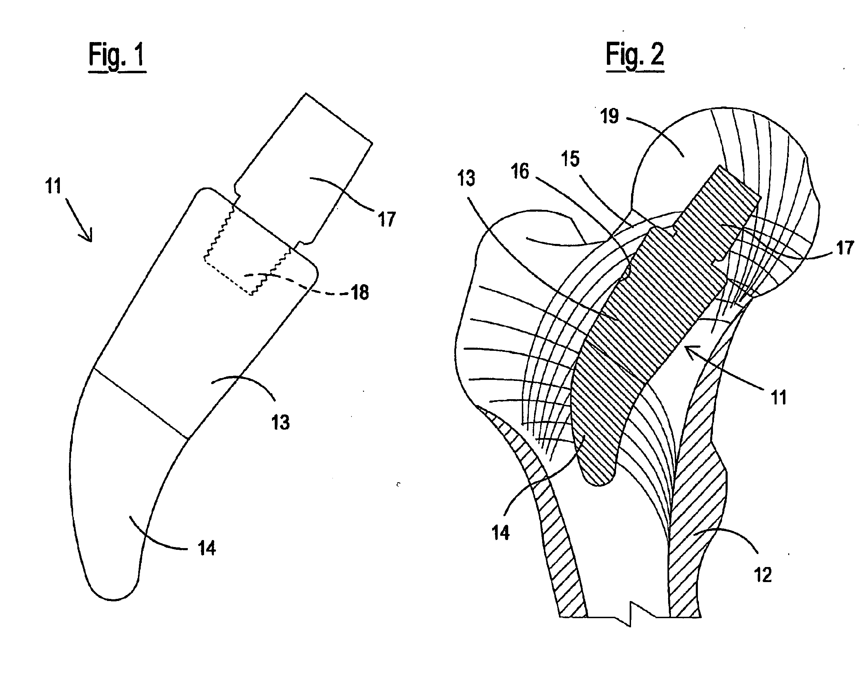

With reference at first to figure 1, it shows a schematic view of a femoral neck prosthesis not falling under the invention denoted as a whole by 11.

The prosthesis 11 is a femoral neck prosthesis, advantageously developed by considering the biomechanics of the femur 12, while pursuing the aim of being minimally invasive, bone saving, satisfactory for the patient.

As it can be noted in the figures it comprises a frustoconical body portion 13 extending in a squat and curved terminal end or tail 14.

In more detail the frustoconical body portion 13 has a variable length depending on the physical characteristics of the patient, but due to its position and function it has a length substantially ranging from 3 to 4 cm.

The diameter of the larger and smaller bases of the frustum of cone also changes depending on the physical characteristics of the patient, however it can be stated that generally such diameters range from 14 to 28 mm for the smaller base and from 16 to 30 mm for the larger one.

Such dimensions allow the prosthesis to be fitted at the calcar region but not to be used instead of a conventional hip prosthesis stem to be fitted into the medullary canal of the femur; it has to be noted that the latter in addition to be different as regards dimensions and shape is also clearly different as regards the applied forces and as regards the way they are released on the femur, such that the two types of prostheses have to be considered as being completely different one another.

With reference now to the tail 14 this is squat, meaning that its length does not exceed the length of the frustoconical body 13, but its length, projected on the axis of the body 13, particularly ranges from 50% to 75% of the body 13.

The tail 14 is conical, meaning that it starts with the same diameter as the smaller base of the body 13 while ending with a rounded tip, having a smaller diameter.

The larger diameter and that of the tip are joined together in a continuous manner, such to prevent any steps to be made which otherwise would cause an area with a high concentration of load to be generated, which is absolutely non physiologic. Still for the same reason, the body 13 and the tail 14 are connected together without interruption (expect for the thickness of the coating, 500 micrometres), thus the outer surface of the prosthesis 11, in the area where the frustoconical body 13 and the curved tail 14 are connected together, can be considered continuous.

The radius of curvature also changes depending on the physical characteristics of the patient, but it ranges approximately from 30 mm to 50 mm.

Thanks to the characteristics outlined above the prosthesis 11 is particularly suitable for being fitted in position with the body 13 filling the neck 15 of the femur 12.

Advantageously the frustoconical body 13 is rough, with a roughness Rz changing in a range from 100 to 300 micrometres, while the tail 14 is smooth, with a roughness Ra less than 0,1 micrometres.

Even if in principle it would be possible to roughen the tail 14, this is however not advisable since this would make hard to properly place the prosthesis 11 and it would cause the implant to be subjected to osseointegration even in the region of the curved tail which on the contrary has to be used only for releasing forces.

The osseointegration in this region, due to the principles that have led to the production of the device (which does not search for a support and therefore for a firm connection with cortical walls), is not strictly necessary for the stability of the implant.

On the contrary the curved and smooth shape of the tail 14 guarantees the forces transmitted to the bone trabeculae to be physiologically released without interfering with the cortical walls and it allows an excellent anti-rotation feature to be obtained.

A series of advantages arise from that: firstly the prosthesis is fitted in position in a simple way due to the smooth tip and it is firmly fixed on the femoral bone 12 both due to the shape of the body 13 and to the roughness thereof, which improves the adhesion with the femur 12 increasing the coefficient of friction one with the other, such that the anchorage to the bone is optimized, and the prosthesis can be implanted without requiring bone cement.

Such arrangement improves also the anti-rotation feature of the prosthesis 11.

The latter is prevented from rotating into the femur 12 thanks to two different factors: firstly the curved tail 14 interferes with the seat where it is housed and it prevents an undesired rotation to be made thanks to its shape.

Secondly the body 13 provided with a high roughness (in addition to improve the introduction by interference as just described) further increases also the coefficient of friction with the seat 16 of the femur 12, thus it has an anti-rotation feature by interference as well, such that it is possible to say that in this base embodiment also the body 13 is provided with anti-rotation means.

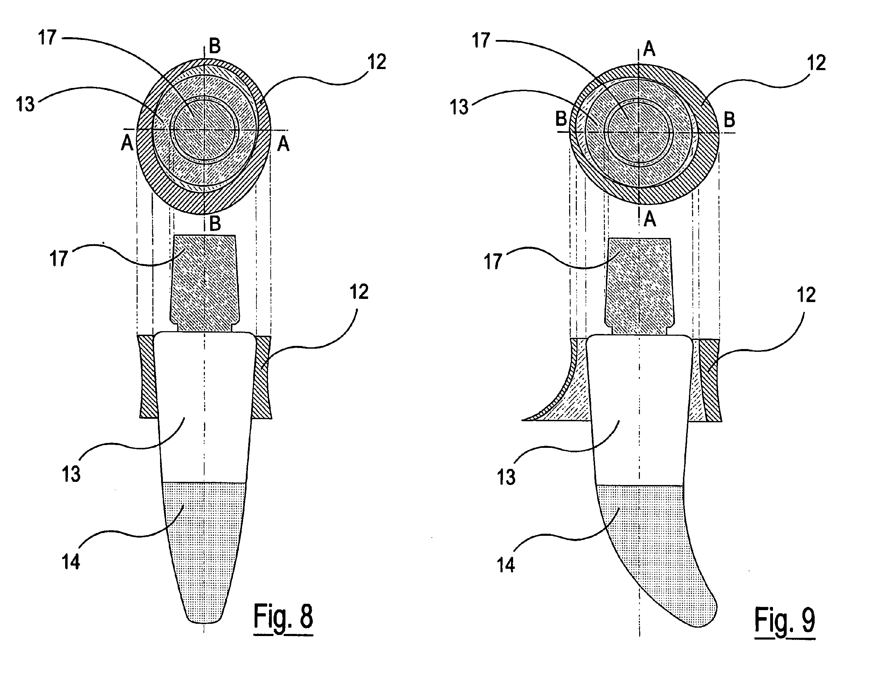

Such feature is clear by examining figures 8 and 9: the seat 16 into the femur 12 obviously does not have a perfect circular shape, but it has a typical oval shape.

The circular body 13 is received therein, and due to the different shape, it is in contact only with a part of the whole seat of the femur 12, as it can be seen in the two planes AA e BB of figures 8 and 9.

Then it is understood that in order to help the tail 14 in preventing the prosthesis 11 from rotating on its axis, it is important for the body 13 to be also provided with anti-rotation means, that in this case consist of the roughened surface of the body, which is in contact with the femur 12, even if partially: it is appropriate, in this way, to try to maximize the coefficient of friction with the latter, the contact areas being small.

The above shows how the prosthesis 11 comprises a frustoconical body 13 provided with first and second anti-rotation means: the first anti-rotation means consist of the curved tail 14 and the second anti-rotation means in this example consist of the roughness of the surface of the body.

As it will be shown below, with reference to the description of figures 10-15, such anti-rotation means can consist, as a combination or as an alternative, even of further solutions, such as for example non circular shapes of the body 13 or of a part thereof or even interference tabs.

The portion of the frustoconical body 13 can be coated or be as one piece with the tail 14 and it serves for filling the neck 15 of the femur 12, as shown in figure 2.

The body 13 and the tail 14 are preferably as one piece one with the other and the body 13 is roughened by a suitable surface treatment of the body.

As an alternative, in the case the body is coated, such coating is composed of 2 layers applied by controlled atmosphere plasma spraying process. The first layer in contact with the body 13 consists of a thickness of 500 micrometres made of porous pure Titanium and it serves for increasing the coefficient of friction in order to give immediate stability; the second layer consists of a thickness of 50 micrometres made of hydroxyapatite and it serves for promoting osseointegration that is the ingrowth process of the bone which becomes attached to the device resulting in a long term stability. The application technology makes such double coatings integral with the body 13, in order to prevent them from move one with respect to the other.

Thanks to its shape it is arranged into a conical seat 16 of the neck 15 of the femur 12, once the femoral head 19 has been resected (by subcapital resection).

Thus the frustoconical portion 13 allows it to be fastened by press-fit, that is by interference fit, such to obtain a high primary and secondary stability of the implant.

The tail 14 is squat, curved and smooth and it essentially serves for centring the prosthesis 11 in the femur 12. Moreover its shape gives a high rotational stability, as mentioned above, and it makes a biomechanical symbiosis with the bone trabeculae of the femoral proximal area.

The frustoconical portion 13 and the squat and curved tail 14 can be made as one piece or as two pieces made of different material, firmly joined together in order to promote the firm positioning of the prosthesis 11 into the femur and to prevent the parts from rotating.

Therefore there is provided a femoral neck prosthesis whose peculiarity is a particular "double section" geometric configuration.

The frustoconical portion is solid, it optimally fills neck 15 of the femur and it exploits the whole support on the calcar.

Moreover the squat, smooth and curved portion 14 serves for "sliding" on the bone trabeculae of the femoral neck 15, by being guided along the lines of force upon the insertion. This "tail" portion 14 is considerably important since it interacts with the bone trabeculae thus supporting the portion 14 as an arc can do with its keystone, supporting the whole prosthesis 11.

The prosthesis 11 further comprises a second frustoconical element 17, or connection terminal, which tapers in a direction opposite to the one of the body 13, thus the larger bases of the two frustums of cone of the body 13 and of the connection terminal 17 are faced each other.

Advantageously, the connection terminal 17 is a so called "12-14" cone with 50°42'30 " summit angle.

Such name identifies a type of cone universally known and used on most of the prosthetic stems.

However as an alternative it is possible to provide other types of the same kind of connection terminal, for example with different dimensions.

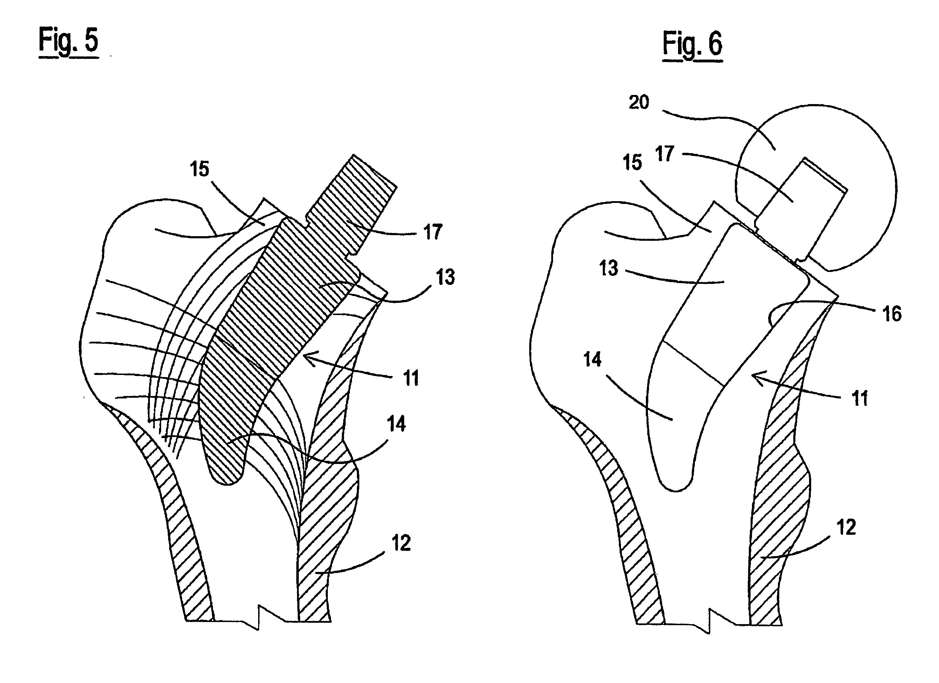

In figure 6 it can be seen that the connection terminal 17, arranged on the free end of the frustoconical portion 13, is coupled to a relevant hemispherical element 20 to restore the head of the femur.

The connection of the hemispherical element 20 and of the terminal 17 is made by a mutual interference, the former being provided with a conical seat with the frustum of cone of the terminal closely fitting therein, this is called "morse taper connection".

Thus, the lines of force are restored which give compactness and stability to the whole implant.

Obviously other types of connections are possible, for example by cements, adhesives, screw connections or the like.

The connection terminal 17 may be as one piece with the prosthesis 11 or can be associated thereto for example by a threaded spigot, denoted by 18.

While in the shown prosthesis it has a frustoconical shape, generally it can also have different dimensions or shapes.

Once the prosthesis has been learned the method for positioning the prosthesis 11 into the neck 15 of the femur 12 results therefrom.

The method essentially consists of a phase wherein the head of the femur is resected in the neck 15 of the femur 12. Then there is a phase in which a frustoconical seat 16 is made in the resected region of the neck 15 in which seat 16 the frustoconical portion 13 of the prosthesis 11 is fitted.

The connection between the parts is an interference fit or press-fit obtained by introducing also the squat and curved tail 14 into the femur 12.

It is noted that according to the invention both the frustoconical portion 13 of the prosthesis 11 and the squat and curved tail 14 are biologically fixed in place into the femur 12.

Such prosthesis hasbeen designed and made from biomechanical concepts in order to guarantee its stability and fixation over time.

Advantageously it is noted that the prosthesis is cementless, is not screwed and it does not search for a support on the side cortical. It is well-known that all these bone anchorage systems do not observe the biomechanics and, even if at the beginning they can show a certain stability, they are destined to fail over time.

With reference to figures 10 and 11 there is shown a first embodiment of the prosthesis according to the invention, generally de-noted by 11A and shown according to two perpendicular planes XX and YY cutting the femur 12.

The prosthesis 11A has the same characteristics of the prosthesis 11 therefore they are not further described; like numbers denote like parts with the same characteristics and functions.

It has to be noted that in this prosthesis the second anti-rotation means, that is those associated to the body 13 comprise also a plurality of interference tabs 25 radially projecting from the surface of the body 13.

Such tabs 25, in the shown example, are three, but they can be also two, four or more.

Likewise, they can be equally spaced on the outer surface of the body 13 or can be placed one near the other or one away from the other, depending on the needs.

Tabs 25, projecting from the body 13, improve the interference of the latter with the femur 12, such to promote in preventing the prosthesis 11 A from rotating on its axis, for example due to the walking action.

Even in this case it is suitable for the surface of the body 13 and the tail 14 to have the roughness parameters Rz and Ra mentioned above, in order to improve the stability of the implant.

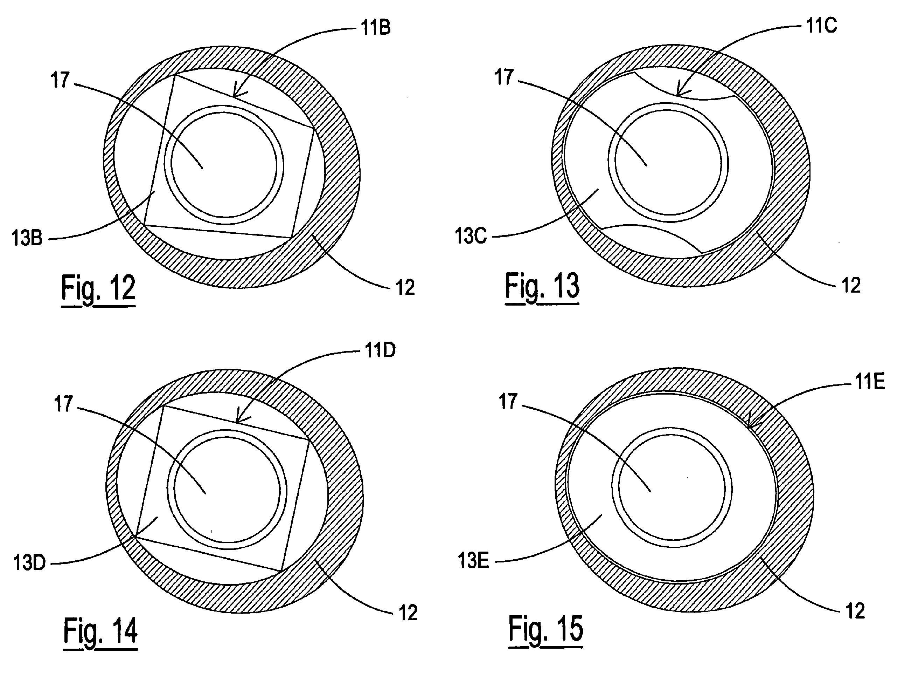

It is also possible to provide further variants for the second anti-rotation means, some of which are shown by way of example in annexed figures 12-15 which do not fall under the invention.

The latter show four variants 11B, 11C, 11D, 11E of the prosthesis described above.

In these variant like numbers denote like parts, therefore they are not further described, but it should be reminded that the same characteristics of the prosthesis 11 are likewise provided.

The difference between the prosthesis 11 and the variants 11B, 11C, 11D, 11E consists mainly in the second anti-rotation means, which in such variants consists of an asymmetrical shape of the body 13B, 13C, 13D, 13E.

While the body 13 had a frustoconical shape with frustums of cone with a circular shape, in this case the frustoconical shape is obtained with bases of different shapes, for example a regular or irregular polygonal shape, such as in 13B and 13D, or a base with a substantially oval shape, such as the case of the body 13E, or even a base unsymmetrical with respect to at least a plane, such as the case of the body 13C, oval with two notches.

It is clear that, in this way, the person skilled in the art has many choices and these mainly depend on implantation requirements on different patients.

Thus the general aim mentioned in the preamble of the description is achieved while overcoming all the drawbacks of the described prior art.

Obviously, the shapes of the structure for making a femoral neck prosthesis of the invention can be different from those shown purely by way of non limitative example in the drawings, the materials and the assembling methods can be different as well.

The field of protection of the invention therefore is delimited by the annexed claims.

## Claims

1. Femoral neck prosthesis (11, 11A, 11B, 11C, 11D, 11E) comprising
- a frustoconical body (13, 13B, 13C, 13D, 13E) positionable into a seat (16) of a neck (15) of a femur (12),
- a curved tail (14), extending from an end of said frustoconical body (13, 13B, 13C, 13D, 13E)
- first and second anti-rotation means in order to prevent said prosthesis (11, 11A, 11B, 11C, 11D, 11E) from rotating on its axis in the operating condition,
said first anti-rotation means being substantially coinciding with said curved tail (14) and said second anti-rotation means being provided on said body (13, 13B, 13C, 13D, 13E);
**characterized in that** said second anti-rotation means comprise a plurality of interference tabs (25) radially projecting from the surface of said body (13).

2. Prosthesis (11, 11A, 11B, 11C, 11D, 11E) according to claim 1, **characterized in that** said tail (14) has an outer surface having a surface roughness Ra less than 0,1 micrometres.

3. Prosthesis (11, 11A, 11B, 11C, 11D, 11E) according to one or more claims 1 to 2, **characterized in that** said body (13, 13B, 13C, 13D, 13E) and said tail (14) are connected together without interruption.

4. Prosthesis (11, 11A, 11B, 11C, 11D, 11E) according to one or more claims 1 to 3, **characterized in that** said prosthesis (11, 11A, 11B, 11C, 11D, 11E) further comprises a frustoconical connection terminal (17), which tapers in a direction opposite to that of said body (13, 13B, 13C, 13D, 13E), said connection terminal (17) being connectable to a hemispherical element (20) for restoring a femoral head, wherein the connection of said hemispherical element (20) and of said terminal (17) is made by a mutual interference, the former being provided with a conical seat wherein the latter is fitted and with which the latter interferes.

5. Prosthesis (11, 11A, 11B, 11C, 11D, 11E) according to one or more of the preceding claims, **characterized in that** said second anti-rotation means consist in an unsymmetrical shape of said body (13B, 13C, 13D, 13E).

## Patentansprüche

1. Femurhalsprothese (11, 11A, 11 B, 11C, 11D, 11E), umfassend:
- einen kegelstumpfförmigen Körper (13, 13B, 13C, 13D, 13E), der in einem Sitz (16) eines Halses (15) eines Femurs (12) positionierbar ist,
- einen gekrümmten Schaft (14), der sich von einem Ende des kegelstumpfförmigen Körpers (13, 13B, 13C, 13D, 13E) erstreckt,
- ein erstes und zweites Drehsicherungsmittel, um die Prothese (11, 11A, 11B, 11C, 11D, 11E) an einem Drehen an ihrer Achse in dem Betriebszustand zu hindern,
wobei das erste Drehsicherungsmittel im Wesentlichen mit dem gekrümmten Schaft [14) übereinstimmt und das zweite Drehsicherungsmittel an dem Körper (13, 13B, 13C, 13D, 13E) vorgesehen ist; dadurch **gekennzeichne**t, dass das zweite Drehsicherungsmittel eine Mehrzahl von Überlagerungslaschen (25) umfasst, die radial von der Oberfläche des Körpers (13) vorragen.

2. Prothese (11, 11A, 11B, 11C, 11D, 11E) nach Anspruch 1,
**dadurch gekennzeichnet, dass** der Schaft (14) eine Außenfläche mit einer Oberflächenrauheit Ra von kleiner als 0,1 Mikrometer besitzt.

3. Prothese (11, 11A, 11B, 11C, 11D, 11E) nach einem der Ansprüche 1 bis 2,
**dadurch gekennzeichnet, dass** der Körper (13, 13B, 13C, 13D, 13E) und der Schaft (14) ohne Unterbrechung miteinander verbunden sind.

4. Prothese (11, 11A, 11B, 11C, 11D, 11E) nach einem der Ansprüche 1 bis 3,
**dadurch gekennzeichnet, dass** die Prothese (11, 11A, 11B, 11C, 11D, 11E) ferner einen kegelstumpfförmigen Verbindungsanschluss (17) umfasst, der sich in einer Richtung verjüngt, die der des Körpers (13, 13B, 13C, 13D, 13E) entgegengesetzt ist, wobei der Verbindungsanschluss (17) mit einem halbkugelförmigen Element (20) zum Wiederherstellen eines Femurkopfes verbindbar ist, wobei die Verbindung des halbkugelförmigen Elements (20) und des Anschlusses (17) durch eine gegenseitige Überlagerung hergestellt ist, wobei das erstere mit einem konischen Sitz versehen ist, in den der letztere eingepasst ist und mit dem sich der letztere überlagert.

5. Prothese (11, 11A, 11B, 11C, 11D, 11E) nach einem der vorhergehenden Ansprüche,
**dadurch gekennzeichnet, dass** das zweite Drehsicherungsmittel in einer unsymmetrischen Form des Körpers (13B, 13C, 13D, 13E) vorhanden ist.

## Revendications

1. Prothèse de col du fémur (11,11A, 11B, 11C, 11D, 11E) comprenant un corps tronconique (13, 13B, 13C, 13D, 13E) pouvant être positionné dans un logement (16) d'un col (15) de fémur (12),
une queue courbe (14), s'étendant à partir d'une extrémité dudit corps tronconique (13,13B, 13C, 13D, 13E),
des premier et second moyen anti-rotation destinés à empêcher ladite prothèse (11, 11A, 11B, 11C, 11D, 11E) de tourner sur son axe lors du fonctionnement,
ledit premier moyen anti-rotation coïncidant sensiblement avec ladite queue courbe (14) et ledit second moyen anti-rotation étant prévu sur ledit corps (13,13B,13C,13D,13E) ;
**caractérisée en ce que** ledit second moyen anti-rotation comporte une pluralité de pattes d'ajustement (25) dépassant radialement depuis la surface dudit corps (13).

2. Prothèse (11, 11A, 11B, 11C, 11D, 11E) selon la revendication 1, **caractérisée en ce que** ladite queue (14) comporte une surface extérieure ayant une rugosité de surface Ra inférieure à 0,1 micron.

3. Prothèse (11, 11A, 11B, 11C, 11D, 11E) selon l'une des revendications 1 à 2, **caractérisée en ce que** ledit corps (13, 13B, 13C, 13D, 13E) et ladite queue (14) sont reliés l'un à l'autre sans interruption.

4. Prothèse (11, 11A, 11B, 11C, 11D, 11E) selon l'une des revendications 1 à 3, **caractérisée en ce que** ladite prothèse (11, 11A, 11B, 11C, 11D, 11E) comprend en outre une tête de raccordement tronconique (17), qui s'effile dans une direction opposée à celle dudit corps (13, 13B, 13C, 13D, 13E), ladite tête de raccordement (17) pouvant être reliée à un élément hémisphérique (20) pour reconstituer une tête fémorale, dans laquelle le raccordement dudit élément hémisphérique (20) et de ladite tête (17) est effectué par un ajustement mutuel, le premier étant muni d'un logement conique dans lequel la seconde est insérée et avec lequel elle forme un ajustement serré.

5. Prothèse (11, 11A, 11B, 11C, 11D, 11E) selon l'une ou plusieurs des précédentes revendications, **caractérisée en ce que** ledit second moyen anti-rotation consiste en une forme asymétrique dudit corps (13B, 13C, 13D, 13E).
